# EUROPEAN PATENT APPLICATION

(11) **EP 2 769 724 A1**
(43) Date of publication of application: **27.08.2014**
(21) Application number: 13305198.7
(22) Date of filing: 22.02.2013
(51) Int. Cl.: A61K 31/575, A61P 29/00

(54) **Inhibitors of Na(v) 1.9 channel activity and uses thereof for treating pain**

(71) Applicant: Université d'Aix-Marseille, 13007 Marseille (FR); Centre National de la Recherche Scientifique, 75016 Paris (FR)
(72) Inventor: Roubinet, Françoise Marie-Dominique, 13530 TRETS (FR); Delmas, Patrick, 05190 REMOLLON (FR); Amsalem, Muriel, Edith, 13127 VITROLLES (FR)
(74) Representative: Starck-Loudes, Anne-Caroline

(57) **Abstract**

The present invention relates to novel compounds for use for preventing, alleviating or treating pain in a subject. Also herein described are pharmaceutical compositions, their preparation and uses as well as methods for preventing, alleviating or treating pain using such compounds and compositions.

## Description

The present invention relates to novel compounds for use for preventing, alleviating or treating pain in a subject. Also herein described are pharmaceutical compositions, their preparation and uses as well as methods for preventing, alleviating or treating pain using such compounds and compositions.

### TECHNICAL BACKGROUND

Pain is the main reason for visiting the emergency department in more than 50% of cases (Cordell WH, Keene KK, Giles BK, Jones JB, Jones JH, Brizendine EJ. The high prevalence of pain in emergency medical care. American Journal of Emergency Medicine. 2002;20(3):165-9.) and is present in 30% of family practice visits (Hasselström J, Liu-Palmgren J, Rasjö-Wrååk G. Prevalence of pain in general practice. Eur J Pain. 2002;6(5):375-85.). Several epidemiological studies from different countries have reported widely varying prevalence rates for chronic pain, ranging from 12-80% of the population (Abu-Saad Huijer H. Chronic pain: a review. J Med Liban. 2010;58(1):21-7). It becomes more common as people approach death. A study of 4,703 patients found that 26% had pain in the last two years of life, increasing to 46% in the last month (Smith AK, Cenzer IS, Knight SJ, Puntillo KA, Widera E, Williams BA, Boscardin WJ, Covinsky KE. The epidemiology of pain during the last 2 years of life. Ann. Intern. Med.. 2010;153(9):563-9.).

Treatment of pain includes the use of local anesthetics, which block neuronal transmission and affect sensation as well as pain, and analgesics, which relieve pain and additionally may interfere with the activity of chemical mediators of inflammation.
Acute pain is usually managed with medications such as analgesics and anesthetics. Management of chronic pain, however, is much more difficult and may require the coordinated efforts of a pain management team, which typically includes medical practitioners, clinical psychologists, physiotherapists, occupational therapists, physician assistants, and nurse practitioners (Thienhaus, O; Cole, BE (2002). "The classification of pain". In Weiner, RS. Pain management: A practical guide for clinicians. American Academy of Pain Management. p. 29. ISBN 0-8493-0926-3.).
An analgesic (also known as a painkiller) is any member of the group of drugs used to relieve pain (achieve analgesia). Their effectiveness relies on how they are able to block the nerve messages that are sent by the pain receptors to the brain. They further have an effect on the body temperature to increase it (known as fever) or to decrease it (Neuss, G. (2007). Chemistry: Course Companion. Oxford: Oxford University Press. p. 628).

Analgesic drugs act in various ways on the peripheral and central nervous systems; they include paracetamol (para-acetylaminophenol, also known in the US as acetaminophen or simply APAP), the non-steroidal anti-inflammatory drugs (NSAIDs) such as the salicylates, and opioid drugs such as morphine and opium. They are distinct from anesthetics, which reversibly eliminate sensation.
In choosing analgesics, the severity and response to other medication determines the choice of agent; the World Health Organization (WHO) pain ladder, originally developed in cancer-related pain, is widely applied to find suitable drugs in a stepwise manner (Anonymous (1990). Cancer pain relief and palliative care; report of a WHO expert committee. World Health Organization Technical Report Series, 804. Geneva, Switzerland: World Health Organization. pp. 1-75). It is a guideline on how pain should be managed. The first step contains the mild analgesics, such as aspirin, ibuprofen or paracetamol. If the pain persists or increases, then the patients should be treated with analgesics in the second step, which are weak opioids like codeine. If the pain still persists, then strong opioids should be used, such as morphine (Neuss, G. (2007). Chemistry: Course Companion. Oxford: Oxford University Press. pp. 628). The analgesic choice is also determined by the type of pain: for neuropathic pain, traditional analgesics are less effective, and there is often benefit from classes of drugs that are not normally considered analgesics, such as tricyclic antidepressants and anticonvulsants (Dworkin RH, Backonja M, Rowbotham MC, et al. (2003). "Advances in neuropathic pain: diagnosis, mechanisms, and treatment recommendations". Arch. Neurol. 60 (11): 1524-34.. Arch. Neurol. 60 (11): 1524-34.).
The exact mechanism of action of paracetamol/acetaminophen is uncertain, but it appears to be acting centrally rather than peripherally (in the brain rather than in nerve endings). Aspirin and the other non-steroidal anti-inflammatory drugs (NSAIDs) inhibit cyclooxygenases, leading to a decrease in prostaglandin production. This reduces pain and also inflammation (in contrast to paracetamol and the opioids).
Paracetamol has few side effects and is regarded as safe, although intake above the recommended dose can lead to liver damage, which can be severe and life-threatening, and occasionally kidney damage. While paracetamol is usually taken orally or rectally, an intravenous preparation introduced in 2002 has been shown to improve pain relief and reduce opioid consumption in the perioperative setting.
NSAIDs predispose to peptic ulcers, renal failure, allergic reactions, and occasionally hearing loss, and they can increase the risk of hemorrhage by affecting platelet function. The use of aspirin in children under 16 suffering from viral illness has been linked to Reye's syndrome, a rare but severe liver disorder.
Morphine, the archetypal opioid, and various other substances (e.g. codeine, oxycodone, hydrocodone, dihydromorphine, pethidine) all exert a similar influence on the cerebral opioid receptor system. Buprenorphine is thought to be a partial agonist of the opioid receptor, and tramadol is an opiate agonist with SNRI properties. Tramadol is structurally closer to venlafaxine than to codeine and delivers analgesia by not only delivering "opiate-like" effects (through mild agonism of the mu receptor) but also by acting as a weak but fast-acting serotonin releasing agent and norepinephrine reuptake inhibitor (Driessen B, Reimann W (January 1992). "Interaction of the central analgesic, tramadol, with the uptake and release of 5-hydroxytryptamine in the rat brain in vitro". British Journal of Pharmacology 105 (1): 147-51.). Dosing of all opioids may be limited by opioid toxicity (confusion, respiratory depression, myoclonic jerks and pinpoint pupils), seizures (tramadol), but there is no dose ceiling in patients who accumulate tolerance.
Flupirtine is a centrally acting K⁺ channel opener with weak NMDA antagonist properties (Kornhuber J, Bleich S, Wiltfang J, Maler M, Parsons CG (1999). "Flupirtine shows functional NMDA receptor antagonism by enhancing Mg2+ block via activation of voltage independent potassium channels. Rapid communication". J Neural Transm 106 (9-10): 857-67.) It is used in Europe for moderate to strong pain and migraine and its muscle relaxant properties. It has no anticholinergic properties and is believed to be devoid of any activity on dopamine, serotonin or histamine receptors. It is not addictive and tolerance usually does not develop (Klawe, C; Maschke, M (2009). "Flupirtine: pharmacology and clinical applications of a nonopioid analgesic and potentially neuroprotective compound". Expert opinion on pharmacotherapy 10 (9): 1495-500.). However, tolerance may develop in single cases (Stoessel C, Heberlein A, Hillemacher T, Bleich S, Kornhuber J (August 2010). "Positive reinforcing effects of flupirtine-two case reports". Prog. Neuropsychopharmacol. Biol. Psychiatry 34 (6): 1120-1.).
In patients with chronic or neuropathic pain, various other substances may have analgesic properties. Tricyclic antidepressants, especially amitriptyline, have been shown to improve treatment of pain in what appears to be a central manner. Nefopam is used in Europe for pain relief with concurrent opioids. The exact mechanism of carbamazepine, gabapentin and pregabalin is similarly unclear, but these anticonvulsants are used to treat neuropathic pain with differing degrees of success. Anticonvulsants are most commonly used for neuropathic pain as their mechanism of action tends to inhibit pain sensation.
Analgesics are frequently used in combination, such as the paracetamol and codeine preparations found in many non-prescription pain relievers. They can also be found in combination with vasoconstrictor drugs such as pseudoephedrine for sinus-related preparations, or with antihistamine drugs for allergy sufferers.
While the use of paracetamol, aspirin, ibuprofen, naproxen and other NSAIDS concurrently with weak to mid-range opiates (up to about the hydrocodone level) has been said to show beneficial synergistic effects by combating pain at multiple sites of action (Mehlisch DR (2002). "The efficacy of combination analgesic therapy in relieving dental pain". J Am Dent Assoc 133 (7): 861-71.), several combination analgesic products have been shown to have few efficacy benefits when compared to similar doses of their individual components. Moreover, these combination analgesics can often result in significant adverse events, including accidental overdoses, most often due to confusion which arises from the multiple (and often non-acting) components of these combinations (Mumion B. "Combination analgesics in adults". Australian Prescriber (33): 113-5. http://www.australianprescriber.com/magazine/33/4/113/5. Retrieved 12 August 2010.).

Inadequate treatment of pain is widespread throughout surgical wards, intensive care units, accident and emergency departments, in general practice, in the management of all forms of chronic pain including cancer pain, and in end of life care. This neglect is extended to all ages, from neonates to the frail elderly. African and Hispanic Americans are more likely than others to suffer needlessly in the hands of a physician; and women's pain is more likely to be undertreated than men's (Hoffmann DE, Tarzian AJ. The girl who cried pain: a bias against women in the treatment of pain. J Law Med Ethics. 2001;29(1):13-27. PMID 11521267). Improved treatments of pain are up to date still highly requested by patients in particular when considering inflammatory and/or chronic pains for which treatment remains incomplete whatever the selected known analgesic molecule.

### SUMMARY OF THE INVENTION

Inventors now herein provide a new solution to efficiently manage pain, for example acute pain, sub-acute pain, chronic pain, allodynia, hyperalgesia, partially treated pain, as well as refractory pains, while avoiding deleterious side effects, in particular gastrointestinal and renal deleterious side effects. In a preferred embodiment, the solution is to efficiently manage inflammatory pain, in particular inflammatory chronic pain; mechanical heat or cold sensitivity (or pain), typically hypersensitivity; visceral pain, in particular an enteric pain, more particularly a gastrointestinal pain or a pain associated with a gastrointestinal syndrome; and/or somatic pain, for example a neuralgia, in particular a trigeminal neuralgia.

This solution involves the use of a product inhibiting the Naᵥ1.9 channel activity, preferably a lipid inhibiting the Naᵥ1.9 channel activity and/or an inhibitor of the degradation of said lipid, for use as active(s) ingredient(s) for preventing, alleviating or treating pain in a subject.

When a lipid inhibiting the Naᵥ1.9 channel activity is used as an active ingredient for preventing, alleviating or treating pain in a subject, this lipid is preferably selected from cholesterol, a sphingolipid and any combination thereof, and is even more preferably soluble cholesterol. Without wishing to be bound by a particular theory, inventors believe that such a lipid is capable of activating a new analgesic pathway by modulating ion channels controlling the excitability of nociceptive neurons.

Another solution involves the use, instead of the previously mentioned lipid or in addition to or in combination with said lipid, typically as an additional active compound, of an inhibitor of the degradation of a lipid inhibiting the Naᵥ1.9 channel activity, preferably of a compound selected from a cholesterol oxidase inhibitor and a sphingomyelinase enzyme.
In a particular embodiment, the product is a combination of a lipid inhibiting the Naᵥ1.9 channel activity and of an inhibitor of the degradation of a lipid inhibiting the Naᵥ1.9 channel activity.

The present invention further relates to i) a composition, typically a pharmaceutical composition, comprising at least such a product, typically a lipid inhibiting the Naᵥ1.9 channel activity and/or an inhibitor of the degradation of said lipid, as an active ingredient and, preferably a pharmaceutically acceptable carrier and/or at least one additional active distinct compound, as well as to ii) any uses thereof, in particular as a local analgesic or anti-hyperalgesic composition.

In a further aspect, methods for preventing, alleviating or treating pain in a subject are herein described. Typically, such a method comprises a step of administering to a subject in need thereof, or of exposing said subject, to a product or composition as herein described inhibiting the Naᵥ1.9 channel activity, possibly in combination with at least one other distinct active compound.

The present document further describes *in vitro, in vivo,* or *ex vivo* screening methods for identifying an agent that modulates a Naᵥ1.9 channel activity.

A particular method as herein described is an *in vitro* or *ex vivo* screening method for identifying an agent that modulates the Naᵥ1.9 channel activity, wherein said method comprises:
a) exposing or contacting a cell, typically an eukaryotic cell, preferably a neuron, expressing Naᵥ1.9 channels, to a test compound, and
b) detecting a decrease in response to a pain stimulus due to said exposition or contacting,
wherein said decrease identifies said test compound as an analgesic/antihyperalgic agent.

A further herein described method is an *in vitro* or *ex vivo* screening method for identifying an agent that modulates the Naᵥ1.9 channel activity, comprising:
a) exposing or contacting a tissue comprising nerves and Naᵥ1.9 channels, for example a skin-nerve preparation or an artificial tissue, to a test compound, and
b) detecting a decrease in response to a pain stimulus due to said exposition or contacting,
wherein said decrease identifies said test compound as an analgesic/antihyperalgic agent.

The present document also describes an *in vivo* method for identifying an analgesic/antihyperalgic agent, comprising:
a) administering a test compound to an animal, in particular an agent having activity in the previously herein described methods, and
b) detecting in said animal a decrease in response to a pain stimulus due to said administering and wherein said animal preferably retains mechanical sensitivity in physiological condition and
preferably also the ability to respond to non-nociceptive sensory stimuli (typically hot, cold and touch),
wherein said decrease identifies said test compound as an analgesic/antihyperalgic agent.

Another object of the invention is a kit comprising i) a product as herein described inhibiting the Naᵥ1.9 channel activity or a composition comprising such a product, preferably ii) at least one additional distinct active compound efficient against pain, and optionally iii) written instructions for using the kit.

### DETAILED DESCRIPTION OF THE INVENTION

Pain is a well known phenomenon as an indicator of actual or potential injury or tissue damage due to inflammation, ischemia, mechanical or other irritation. In pain science, thresholds are measured by gradually increasing the intensity of a stimulus such as electric current or heat applied to the body. The pain perception threshold is the point at which the stimulus begins to hurt, and the pain tolerance threshold is reached when the subject acts to stop the pain.

In 1994, responding to the need for a more useful system for describing chronic pain, the International Association for the Study of Pain (IASP) classified pain according to specific characteristics: (1) region of the body involved (e.g., abdomen, lower limbs), (2) system whose dysfunction may be causing the pain (e.g., nervous, gastrointestinal), (3) duration and pattern of occurrence, (4) intensity and time since onset, and (5) etiology (Merskey H & Bogduk N. Classification of Chronic Pain. 2 ed. Seattle: International Association for the Study of Pain; 1994, pp. 3-4). This system has been criticized by Clifford J. Woolf (Woolf CJ, Bennett GJ, Doherty M, Dubner R, Kidd B, Koltzenburg M, Lipton R, Loeser JD, Payne R, Torebjork E. Towards a mechanism-based classification of pain?. Pain. 1998; 77(3):227-9.) and others as inadequate for guiding research and treatment. According to Woolf (Woolf CJ. What is this thing called pain?. Journal of Clinical Investigation. 2010;120(11):3742-4.), there are three classes of pain : nociceptive pain, inflammatory pain which is associated with tissue damage and the infiltration of immune cells, and pathological pain which is a disease state caused by damage to the nervous system (neuropathic pain) or by its abnormal function (dysfunctional pain, like in fibromyalgia, irritable bowel syndrome, tension type headache, etc.).
Pain is usually transitory, lasting only until the noxious stimulus is removed or the underlying damage or pathology has healed, but some painful conditions, such as rheumatoid arthritis, peripheral neuropathy, cancer and idiopathic pain (pain that persists after the trauma or pathology has healed, or that arises without any apparent cause), may persist for years. Pain that lasts a long time is called *chronic,* and pain that resolves quickly is called *acute.* Traditionally, the distinction between *acute* and *chronic* pain has relied upon an arbitrary interval of time from onset; the two most commonly used markers being 3 months and 6 months since the onset of pain (Turk DC, Okifuji A. Pain terms and taxonomies of pain. In: Bonica JJ, Loeser JD, Chapman CR, Turk DC, Butler SH. Bonica's management of pain. Hagerstwon, MD: Lippincott Williams & Wilkins; 2001.), though some theorists and researchers have placed the transition from acute to chronic pain at 12 months (Spanswick CC, Main CJ. Pain management: an interdisciplinary approach. Edinburgh: Churchill Livingstone; 2000.). Others apply *acute* to pain that lasts less than 30 days, *chronic* to pain of more than six months duration, and *subacute* to pain that lasts from one to six months (Thienhaus O, Cole BE. Classification of pain. In: Weiner R. Pain management: a practical guide for clinicians. Boca Raton: CRC Press; 2002.). A popular alternative definition of *chronic pain,* involving no arbitrarily fixed durations is "pain that extends beyond the expected period of healing." (Turk, D.C.; Okifuji, A. (2001). "Pain terms and taxonomies". In Loeser, D.; Butler, S. H.; Chapman, J.J. et al.. Bonica's management of pain (3 ed.). Lippincott Williams & Wilkins. pp. 18-25. ISBN 0-683-30462-3.). Chronic pain may be classified as cancer pain or benign (Thienhaus, O.; Cole, B.E. (2002). "Classification of pain". In Weiner, R.S.. Pain management: A practical guide for clinicians (6 ed.). American Academy of Pain Management. ISBN 0-8493-0926-3.).
Nociceptive pain is caused by stimulation of peripheral nerve fibers that respond only to stimuli approaching or exceeding harmful intensity (nociceptors), and may be classified according to the mode of noxious stimulation; the most common categories being "thermal" (heat or cold), "mechanical" (crushing, tearing, etc.) and "chemical" (iodine in a cut, chili powder in the eyes). Nociceptive pain may also be divided into "visceral", "deep somatic" and "superficial somatic" pain. Visceral structures are highly sensitive to stretch, ischemia and inflammation, but relatively insensitive to other stimuli that normally evoke pain in other structures, such as burning and cutting.
Visceral pain is diffuse, difficult to locate and often referred to a distant, usually superficial, structure. It may be accompanied by nausea and vomiting and may be described as sickening, deep, squeezing, and dull. *Deep somatic* pain is initiated by stimulation of nociceptors in ligaments, tendons, bones, blood vessels, fasciae and muscles, and is dull, aching, poorly localized pain. Examples include sprains and broken bones. *Superficial* pain is initiated by activation of nociceptors in the skin or other superficial tissue, and is sharp, well-defined and clearly located. Examples of injuries that produce superficial somatic pain include minor wounds and minor (first degree) bums.
Neuropathic pain is caused by damage or disease affecting any part of the nervous system involved in bodily feelings (the somatosensory system) (Treede RD, Jensen TS, Campbell JN, Cruccu G, Dostrovsky JO, Griffin JW, Hansson P, Hughes R, Nurmikko T, Serra J. Neuropathic pain: redefinition and a grading system for clinical and research purposes. Neurology. 2008;70(18):1630-5.). Peripheral neuropathic pain is often described as "burning," "tingling," "electrical," "stabbing," or "pins and needles" (Paice JA. Mechanisms and management of neuropathic pain in cancer. J. Support Oncol.. 2003;1(2):107-20.)
Psychogenic pain, also called *psychalgia* or *somatoform pain,* is pain caused, increased, or prolonged by mental, emotional, or behavioral factors ("Psychogenic pain - definition from Biology-Online.org" Biology-online.org. Retrieved 2008-11-05). Headache, back pain, and stomach pain are sometimes diagnosed as psychogenic. Sufferers are often stigmatized, because both medical professionals and the general public tend to think that pain from a psychological source is not "real". However, specialists consider that it is no less actual or hurtful than pain from any other source.
People with long term pain frequently display psychological disturbance, with elevated scores on the Minnesota Multiphasic Personality Inventory scales of hysteria, depression and hypochondriasis (the "neurotic triad"). Some investigators have argued that it is this neuroticism that causes acute injuries to turn chronic, but clinical evidence points the other way, to chronic pain causing neuroticism. When long term pain is relieved by therapeutic intervention, scores on the neurotic triad and anxiety fall, often to normal levels. Self-esteem, often low in chronic pain patients, also shows improvement once pain has resolved (Wall PD, Melzack R. The challenge of pain. New York: Penguin Books; 1996.).

Unless more precisely identified, the term "pain", as used herein, indifferently refers to any pain or sensitivity (herein understood as an abnormal sensitivity, i.e. typically as an hypersensitivity), typically any Naᵥ1.9-mediated pain, in particular any pain selected from nociceptive pain, inflammatory pain, pathological pain, neuropathic pain, idiopathic pain, chronic pain, acute pain, subacute pain, thermal pain, mechanical pain, chemical pain, visceral pain, deep somatic pain, superficial somatic pain, somatoform pain, psychogenic pain, and psychalgia pain.

Inflammation is known to be responsible for the sensitization of peripheral sensory neurons, leading to spontaneous pain and invalidating pain hypersensitivity. Acute or chronic pathological tissue inflammation strongly impacts on pain perception by sensitizing peripheral sensory neurons, giving rise to local and incapacitating pain hypersensitivity. Inflammatory mediators are known to enhance nociceptive primary afferent fibers excitability, in part by modifying expression and/or function of ion channels present in nerve endings (Woolf CJ, Costigan M (1999), Transcriptional and posttranslational plasticity and the generation of inflammatory pain. Proc Natl Acad Sci U S A 96: 7723-7730). Voltage-gated sodium channels (VGSCs) play a fundamental role in neuronal excitability as they are directly responsible for initiation and propagation of action potentials, and their implication in different chronic pain disorders, including inflammatory pain, is relatively well established (Lai J, Porreca F, Hunter JC, Gold MS (2004) Voltage-gated sodium channels and hyperalgesia. Annu Rev Pharmacol Toxicol 44: 371-397).
Among the 10 VGSC isoforms, two tetrodotoxin-resistant (TTX-R) channels, Nav1.8 and Nav1.9, are almost exclusively expressed in nociceptors, consistently with a specific involvement in nociceptive pathways (Akopian AN, Sivilotti L, Wood JN (1996) A tetrodotoxin-resistant voltage-gated sodium channel expressed by sensory neurons. Nature 379: 257-262; Persson AK, Black JA, Gasser A, Fischer T, Waxman SG (2010).
The Naᵥ1.9 channel, also herein identified as "Naᵥ1.9 channel" or "Naᵥ1.9", is expressed in nociceptive DRG neurons where it contributes to pain behavior after peripheral inflammation. Studies supported a role for the Naᵥ1.9 channel in contributing to pain behavior, in particular to spontaneous pain behavior, and to the persistent heat and mechanical hyperalgesia classically accompanying inflammation (Priest BT, Murphy BA, Lindia JA, Diaz C, Abbadie C, Ritter AM, Liberator P, Iyer LM, Kash SF, Kohler MG, Kaczorowski GJ, Maclntyre DE, Martin WJ. Contribution of the tetrodotoxin-resistant voltage-gated sodium channel NaV1.9 to sensory transmission and nociceptive behavior. Proc Natl Acad Sci U S A. 2005, 102(26):9382-7; Amaya F, Wang H, Costigan M, Allchorne AJ, Hatcher JP, Egerton J, Stean T, Morisset V, Grose D, Gunthorpe MJ, Chessell IP, Tate S, Green PJ, Woolf CJ. The voltage-gated sodium channel Na(v)1.9 is an effector of peripheral inflammatory pain hypersensitivity. J Neurosci. 2006; 26(50):12852-60.). Inventors suggested that this Naᵥ1.9 channel may serve as molecular target for new type of analgesic with minimal side-effect profile (Padilla et al. Expression and localization of the sodium channel in enteric neurons and in trigeminal sensory endings: Implication for intestinal reflex function and orofacial pain. Mol. Cell. Neurosci. 35 (2007) 138-152). Maingret et al. (Inflammatory mediators increase Nav1.9 current and excitability in nociceptors through a coincident detection mechanism. J Gen Physiol. (2008) 131 (3), 211-225) demonstrated that Naᵥ1.9 channel is potentiated by the concerted action of inflammatory mediators that may contribute to nociceptor's hyperexcitability during peripheral inflammation.
More recently, this Naᵥ1.9 sodium channel has been characterized by inventors as not involved in basal pain thresholds but as crucial in the generation of heat and mechanical pain hypersensitivity, both in subacute and chronic inflammatory pain models, and to a lower extent in its maintenance (Lolignier et al., PLoS ONE, August 2011, Vol. 6, Issue 8, e23083).

### Active ingredient

An object of the present invention is a product inhibiting the Naᵥ1.9 channel activity for use as active ingredient for preventing, alleviating or treating pain in a subject, in particular any pain as herein described for example acute pain, sub-acute pain, chronic pain, allodynia, hyperalgesia, partially treated pain, as well as refractory pains, while preferably advantageously avoiding deleterious side effects, in particular gastrointestinal and renal deleterious side effects.
In a preferred embodiment, the product is to efficiently manage inflammatory pain, in particular inflammatory chronic pain; mechanical pain, in particular heat or cold sensitivity (or pain); visceral pain, in particular an enteric pain, for example a gastrointestinal pain or a pain associated with a gastrointestinal syndrome; and/or a somatic pain, for example a neuralgia, in particular a trigeminal neuralgia, for example an ocular pain.
The compounds according to the invention may also be used to prevent or treat chronic pain in subjects suffering from pathologies such as cancer, bums, etc., for which generally analgesics (such as morphine) may be administered for a long period, optionally in delayed form.
The compounds according to the invention may also be used together with reduced daily doses of morphine in order to improve the clinical picture of patients (by limiting side effects of morphinomimetics, such as intestinal disorders, for example).
The product of the invention is in addition advantageous in that it does not hide symptoms associated to the disease responsible for the pain affecting the subject, or associated to an aggravation of said disease, such as in particular fever.

In a particular embodiment, the product of the invention is a lipid inhibiting the Naᵥ1.9 canal activity for use as an active ingredient for preventing, alleviating or treating pain in a subject, in particular any pain as herein described.
The plasma membrane of cells is made of a combination of glycosphingolipids and protein receptors organized in glycolipoprotein microdomains termed lipid rafts (Levitan et al. (2010), review; Thomas S., Pais A.P., Casares S and Brumeanu T.D. (2004). Analysis of lipid rafts in T cells. Molecular Immunology 41: 399-409.). These specialized membrane microdomains compartmentalize cellular processes by serving as organizing centers for the assembly of signalling molecules, influencing membrane fluidity and membrane protein trafficking, and regulating neurotransmission and receptor trafficking (Korade, Z.; Kenworthy, A. K. (2008). "Lipid rafts, cholesterol, and the brain". Neuropharmacology 55 (8): 1265.). Lipid rafts are more ordered and tightly packed than the surrounding bilayer, but float freely in the membrane bilayer (Simons, K.; Ehehalt, R. (2002). "Cholesterol, lipid rafts, and disease". Journal of Clinical Investigation 110 (5): 597.). One key difference between lipid rafts and the plasma membranes from which they are derived is lipid composition. Research has shown that lipid rafts generally contain 3 to 5-fold the amount of cholesterol found in the surrounding bilayer. Also, lipid rafts are enriched in sphingolipids such as sphingomyelin, which is typically elevated by 50% compared to the plasma membrane.
Inventors now herein demonstrate that the Naᵥ1.9 channel is present in lipid rafts. Inventors also surprisingly discovered and herein demonstrate that a lipid depletion, preferably a cholesterol depletion, greatly enhance *in vivo* the Naᵥ1.9 channel activity.
The lipid for use as an active ingredient in the context of the present invention is preferably selected from cholesterol, in particular soluble cholesterol, a sphingolipid, and any combination thereof.

In another embodiment, the active ingredient usable instead of the previously mentioned lipid, or in addition to or combination with, can be advantageously selected from a cholesterol inhibitor and/or a sphingolipid degrading enzyme. An example of such a compound is the GW 4869 compound, which has been described by Luberto and coworkers (Luberto, C., Hassler, D.F., Signorelli, P., et al. (2002) "Inhibition of tumor necrosis factor-induced cell death in MCF7 by a novel inhibitor of neutral sphingomyelinase". J Biol Chem 277(43) 41128-41139).

In a particular embodiment, the lipids of the invention, in particular cholesterol, or the inhibitor of cholesterol and/or sphingolipid degrading enzyme, in addition to Naᵥ1.9 advantageously modulate the activity of at least one other ion channel such as a potassium channel, calcium channel, ion co-transporter and the like, and/or affect the hERG channel or another physiologically relevant channel.

### Subject

In the context of the present invention, the patient or subject is an animal, preferably a vertebrate, typically a mammal. In a preferred embodiment, the mammal is a human being, whatever its age or sex. The mammal may further be animal, in particular a domestic or breeding animal, in particular horses, dogs, etc.
In a particular embodiment, the subject suffers of an inflammatory pain, in particular a chronic inflammatory pain.
In another particular embodiment, the subject is resistant to the applied or to an already tested pain treatment, in particular to paracetamol, or suffers from gastrointestinal and/or renal adverse effects induced for example by aspirin or ibuprofen.

### Compositions

A further object of the invention relates to a pharmaceutical composition comprising a product inhibiting the Naᵥ1.9 channel activity as herein described as an active ingredient, and preferably a pharmaceutically acceptable carrier.

A "pharmaceutical composition" refers to a formulation of a compound of the invention (active ingredient) and a medium generally accepted in the art for the delivery of biologically active compounds to the subject in need thereof. Such a carrier includes all pharmaceutically acceptable carriers, diluents, medium or supports therefore. This carrier can be selected for example from methyl-beta-cyclodextrin, a polymer of acrylic acid (such as carbopol), a mixture of polyethylene glycol and polypropylene glycol, monoetrhanol amine and hydroxymethyl cellulose.

When cholesterol is used as the active ingredient, such a carrier is only optional. When a soluble form of cholesterol is used, methylbetacyclodextrin for example can be used adequately in combination with cholesterol, as the only medium or together with another medium as previously described.

Conventional pharmaceutical practice may be employed to provide suitable formulations or compositions to subjects, for example in unit dosage form.

This composition is typically a local analgesic/anti-hyperalgesic composition.

The products of the invention (lipids, inhibitors of lipid degradation, and compositions) are for use for preventing, alleviating or treating pain in a subject. As explained previously, pain is typically selected from an acute pain; a subacute pain; and a chronic pain. Pain may further be selected from a mechanical sensitivity or pain, for example a heat or cold sensitivity or pain; an inflammatory pain; a visceral pain, in particular an enteric pain, more particularly a gastrointestinal pain or a pain associated with a gastrointestinal syndrome; allodynia; hyperalgesia,; somatic pain, for example a neuralgia, in particular a trigeminal neuralgia (for example an ophthalmic pain); and any refractory pain.
Pain is preferably a chronic and/or inflammatory pain, even more preferably a chronic inflammatory pain.
In a particular embodiment, pain is a pain sensitive to steroidal anti-inflammatory drug (SAID), non steroidal anti-inflammatory drug (NSAID) or opioid, and said pain is to be treated with a combination of the product according to the invention together with a reduced amount of SAID, NSAID or opioid when compared to the administration of SAID, NSAID or opioid alone.

In another embodiment, pain is a pain where one of opioid, ibuprofen or aspirin is classically used and where doses are to be reduced to avoid their side effects, typically to avoid gastrointestinal and/or renal adverse effects.
In another particular embodiment, the subject or its pain itself is resistant to a drug selected from steroidal anti-inflammatory drug (SAID), non steroidal anti-inflammatory drug (NSAID) and opioid, or the subject suffers from gastrointestinal and/or renal adverse effects induced by said drug.
In a particular embodiment, pain is a pain resistant to paracetamol.
The composition of the invention can further comprise at least one additional active compound. This compound can be advantageously selected from a SAID, NSAID or opioid drug. In a preferred embodiment, the additional compound is a drug distinct from the product of the invention also treating or attenuating pain.

In another embodiment, a compound or composition of the invention can also be administered for example along with an agent intended to treat a coincident condition, such as where analgesic and antitumor agents are given together or contemporaneously.

### Treatment

Also herein taught is a method for preventing, alleviating or treating pain in a subject. An aim of the method can be reducing the ion flux activity of the Naᵥ1.9 polypeptide.
A particular method for preventing, alleviating or treating a Naᵥ1.9-mediated pain in a subject in need thereof, comprises administering to the subject an effective amount of a product inhibiting the Naᵥ1.9 channel activity as herein described.
A further particular method for preventing, alleviating or treating pain, typically a Naᵥ1.9-mediated pain, in a subject in need thereof, comprises a step of administering to said subject, or of exposing said subject, to a compound or a composition as herein described inhibiting the Naᵥ1.9 channel activity in a therapeutically effective amount, possibly in combination with at least one other active compound such as any one of the molecules mentioned in the background part, for example aspirin, ibuprofen, paracetamol, opioid, or such as an inhibitor of the degradation of a lipid inhibiting the Naᵥ1.9 channel activity as herein described, preferably selected from a cholesterol oxidase inhibitor and a sphingomyelinase enzym.
In a further aspect, the present description relates to a method for treating a condition in a subject afflicted with a Naᵥ1.9-mediated disease, such as but not limited to a pain as herein described, typically an inflammatory pain, in particular an inflammatory chronic pain, comprising administering to said subject an effective amount of a product of the invention.

As used in this specification "Naᵥ1.9-mediated disease" includes the wide range of disorders, conditions and disease that are caused by, or treatable with, modulation, preferably inhibition, of Naᵥ1.9 channel activity. Such diseases include but are not limited to pain (whether chronic, acute, inflammatory, etc.), neuralgia, neuropathic pain, eudynia, visceral pain, trauma pain, post-operative pain, heat sensitivity, irritable bowel syndrome, Crohn's disease, multiple sclerosis, diabetic neuropathy, arthritic pain, rheumatoid arthritis, sodium channel toxin related illnesses inducing pain, familial rectal pain, cancer and pain associated to chemotherapies, trigeminal neuralgia, migraine headache, and other headaches. In addition, Naᵥ1.9-mediated diseases include such pathologies as inflammatory diseases, neuropathies (e.g., diabetic neuropathy), dystrophies (e.g., reflex sympathetic dystrophy, post-herpetic neuralgia); and trauma (tissue damage by any cause). Inflammatory diseases can include, but are not limited to, visceral inflammatory pathologies and somatic inflammatory pathologies. Acute inflammatory pathologies include, but are not limited to proctite, rectite, and arthritis. Chronic inflammatory pathologies include, but are not limited to sarcoidosis, chronic inflammatory bowel disease, ulcerative colitis, and Crohn's pathology, psoriasis, rosacea, and vascular inflammatory pathologies, such as, but not limited to, disseminated intravascular coagulation, atherosclerosis, and Kawasaki's pathology. In addition, Naᵥ1.9-mediated disease also includes benign prostatic hyperplasia (BPH), hypercholesterolemia, cancer and pruritis. In particular, proctite, Crohn's pathology and ulcerative colitis are identified as visceral pains, arthritis, uveitis psoriasis and rosacea as somatic pains.
As used herein, "disease" includes disorder, condition, symptoms of a disease, incipient disease, anticipated disease or anticipated syndrome, and the like.

### Protocol/Regimen

The compounds or compositions according to the invention may be administered in various ways or route. The product of the invention is preferably for cutaneous, subcutaneous, dermic, transdermic, ocular (for example corneal) or rectal administration to the subject, preferably for topic administration on an inflammation site.

In a preferred embodiment, the lipid of the invention, when used as the active ingredient of one of several active ingredients, is for topic administration, preferably through cutaneous, subcutaneous or transdermic administration to the subject, preferably for topic administration on an inflammation site.

A typical regimen for treatment of Naᵥ1.9-mediated disease comprises administration of an effective amount over a period of one or several days, up to one year, and including between one week and about six months, or it may be chronic. It is understood that the dosage of a pharmaceutical compound or composition of the invention administered *in vivo* will be dependent upon the age, health, sex, and weight of the recipient (subject), kind of concurrent treatment, if any, frequency of treatment, and the nature of the pharmaceutical effect desired. The ranges of effectives doses provided herein are not intended to be limiting and represent preferred dose ranges. However, the most preferred dosage will be tailored to the individual subject, as is understood and determinable by one skilled in the relevant arts (see, e.g., Berkowet et al., eds., The Merck Manual, 16th edition, Merck and Co., Rahway, N.J., 1992; Goodmanetna., eds., Goodman and Cilman's The pharmacological Basis of Therapeutics, 10th edition, Pergamon Press, Inc., Elmsford, N.Y., (2001); Avery's drug treatment: Principles and practice of clinical pharmacology and therapeutics, 3rd edition, ADIS Press, LTD., Williams and Wilkins, Baltimore, MD. (1987), Ebadi, Pharmacology, Little, Brown and Co., Boston, (1985); Osolci et al., eds., Remington's Pharmaceutical Sciences, 18th edition, Mack Publishing Co., Easton, PA (1990); Katzung, Basic and clinical pharmacology, Appleton and Lange, Norwalk, CT (1992)).

The total dose required for each treatment can be administered by multiple doses or in a single dose, preferably as soon as the early symptoms of pain appear, or preventively, for example before or during surgery when needed. The pharmaceutical compound can be administered alone or in conjunction with at least one other pharmaceutical directed to the pathology, or directed to other symptoms of the pathology. Effective amounts of a compound or composition according to the invention are from about 0.1µg to about 100 mg/kg body weight, administered at intervals of 4-72 hours for a period of up to 1 year, and/or any range or value therein, such as 0.0001-1.0, 1-10, 10-50 and 50-100, 0.0001-0.001, 0.001-0.01, 0.01-0.1, 0.1-1.0, 1.0-10, 5-10, 10-20, 20-50, and 50-100 mg/kg, for example between 1 and 100 mg/kg, preferably between 1 and 5mg/kg, for example 1,4, 1,5, 1,6, 1.7, 1,8 or 2 mg/kg, at intervals of 1-4, 4-10, 10-16, 16-24, 24-36, 36-48, 48-72 hours, for a period of 1-14, 14-28, or 30-44 days, or 1-24 weeks, or any range or value therein. The recipients of administration of compounds and/or compositions of the invention can be any subjects as herein defined, preferably humans.

### Formulations/ Concentrations

The compounds or compositions according to the invention may be administered in various forms. Thus, they may be formulated in the form of ointment, gel, paste, liquid solutions, suspensions, tablets, gelatin capsules, capsules, suppository (in particular for pain associated with a gastrointestinal syndrome), powders, nasal drops, or aerosol, preferably in the form of ointment. Methods well known in the art for making formulations are found in, for example, Remington: The Science and Practice of Pharmacy, (19th ed.) ed. A.R. Gennaro AR., 1995, Mack Publishing Company, Easton, PA.
The compounds of the invention are typically administered in the form of ointments, gels, oils, tablets, suppositories, powders, gelatin capsules, capsules, etc., optionally by means of dosage forms or devices that ensure prolonged and/or delayed release. For this type of formulation, an agent such as cellulose, carbonate or starch is advantageously used.
For injections, the compounds are generally packaged in the form of liquid suspensions, which may be injected via syringes or perfusions, for example. In this respect, the compounds are generally dissolved in saline, physiological, isotonic or buffered solutions, etc., compatible with pharmaceutical use and known to the person skilled in the art. Thus, the compositions may contain one or more agents or excipients selected from dispersants, solubilizers, stabilizers, preservatives, etc. Agents or excipients that can be used in liquid and/or injectable formulations are notably methylcellulose, hydroxymethylcellulose, carboxymethylcellulose, polysorbate 80, mannitol, gelatin, lactose, vegetable oils, acacia, etc.
It is understood that the flow rate and/or dose administered may be adjusted by the person skilled in the art according to the patient, the pain observed, the area to be treated, the active product(s) concerned, the mode of administration, etc.
For topical applications, it is preferred to expose the subject to be treated to an effective amount of a pharmaceutical compound or composition according to the invention to target areas, e.g., skin surfaces, mucous membranes, and the like, which are adjacent to the peripheral neurons to be treated. Typically, the compounds are administered at doses that may vary between about 0.0001 mg to about 1 g/kg of body weight of a compound of the invention, more generally between about 1 and 100 mg/kg per application, depending upon the previously mentioned criteria, whether the use is prophylactic or therapeutic and the nature of the topical vehicle employed. A preferred topical preparation is an ointment or gel, wherein about 1 to about 100 mg/kg of active ingredient is used per cc of ointment or gel base.
Furthermore, administration by injection may comprise several (2, 3 or 4) administrations per day, if need be.
In addition, for chronic treatments, delayed or prolonged systems may be advantageous, ensuring the subject effective and long-lasting pain treatment.

### Screening

Further herein described are *in vitro, in vivo,* or *ex vivo* screening methods for identifying an agent that modulates a Naᵥ1.9 canal or channel (typically from mouse, rat or human origin) activity.

A particular method as herein described is an *in vitro* or *ex vivo* screening method for identifying an agent that modulates the Naᵥ1.9 channel activity, wherein said method comprises:
a) exposing or contacting a cell, typically an eukaryotic cell, preferably a neuron, expressing Naᵥ1.9 channels, to a test compound, and
b) detecting a decrease in response to a pain stimulus due to said exposition or contacting,
wherein said decrease identifies said test compound as an analgesic/antihyperalgic agent.

The cell can be a cell naturally expressing the Naᵥ1.9 channel (endogenous expression) or a cell which has been genetically modified to express a specific Naᵥ1.9 channel (exogenous expression).

A further herein described method is an *in vitro* or *ex vivo* screening method for identifying an agent that modulates the Naᵥ1.9 channel activity, comprising:
a) exposing or contacting a tissue comprising nerves and Naᵥ1.9 channels, for example a skin-nerve preparation or an artificial tissue, to a test compound, and
b) detecting a decrease in response to a pain stimulus due to said exposition or contacting,
wherein said decrease identifies said test compound as an analgesic/antihyperalgic agent.

The contacting is typically performed by applying the compound at the tissue surface.
The change in expression is preferably a decreased expression identifying the test compound as an inhibitor of the Naᵥ1.9 wild-type nucleic acid activity usable for preventing, alleviating or treating pain in a subject. The detection of a response to pain stimulus can be performed by recording an electrophysiological change in the nerve or neuron activity, typically a decrease in the nerve or neuron activity.

Preferably, this test compound demonstrates selectivity for the Naᵥ1.9 over other sodium channel subunits.

The present document also describes an *in vivo* method for identifying an analgesic/antihyperalgic agent, comprising:
a) administering a test compound to an animal, in particular an agent having activity in the previously herein described methods, and
b) detecting in said animal a decrease in response to a pain stimulus due to said administering and wherein said animal preferably retains mechanical sensitivity in physiological condition and
preferably also the ability to respond to non-nociceptive sensory stimuli (typically hot, cold and touch),
wherein said decrease identifies said test compound as an analgesic/antihyperalgic agent.

Compounds when considered as having potential therapeutic value are subsequently analyzed using any standard *in vitro* assay or *in vivo* animal model for the disease indication known in the art. These compounds are considered as products of the invention and are usable as herein previously explained.

A further object of the invention is a kit comprising i) a product as herein described inhibiting the Naᵥ1.9 canal activity or a composition comprising such a product, preferably ii) at least one additional distinct active compound efficient against pain, and optionally iii) written instructions for using the kit.

According to a specific embodiment, the invention also relates to a kit that is suitable for the treatment by the methods herein described. These kits comprise i) a product as herein described inhibiting the Naᵥ1.9 canal activity or a composition comprising such a product, typically in the dosages herein indicated, and ii) a second composition containing an analgesic compound, preferably an opiate compound, in dosages generally lowered when compared to those classically prescribed, for a simultaneous, separate or sequential administration, in effective amounts according to the invention.

The figures and examples illustrate the invention without limiting its scope.

### LEGENDS TO THE FIGURES

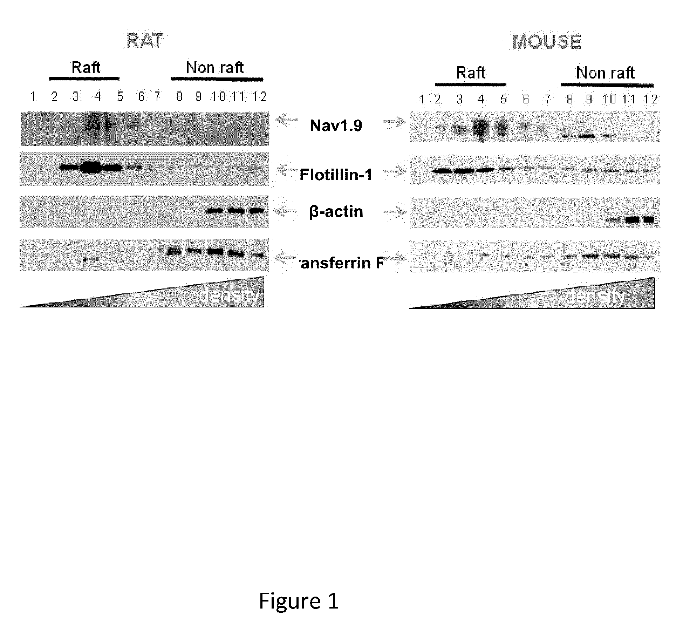

**Figure 1** shows that Nav1.9 concentrates in the flotillin-containing membrane fraction. DRG lysates were fractionated using discontinuous optiprep gradients. Fraction 1 represents the top of the gradient. Gradients were fractionated, and equal amounts of proteins from corresponding fractions were separated by SDS-PAGE. Western blots were probed for Nav1.9, β-actin, flotillin, and TfR1. Flotillin, which is a marker for lipid raft membranes, was used as a marker for the lipid raft fraction, while TfR1and β-actin were used to mark the heavy membrane fractions. These results are representative of at least three independent experiments.

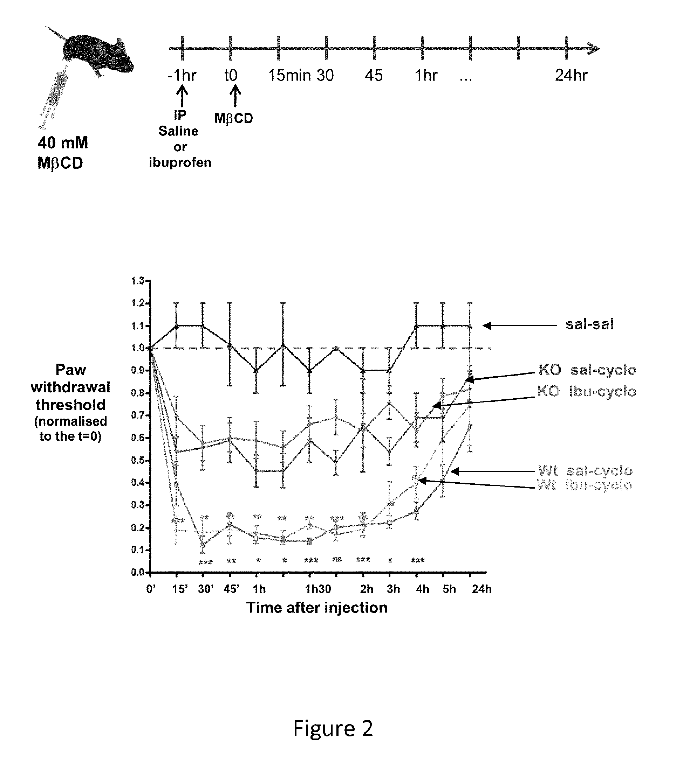

**Figure 2** shows the time course of tactile hyperalgesia measured with von Frey filaments in wild type and Nav1.9 KO mice, intraplantarly injected with 20 µl of methyl-beta-cyclodextrin, (40mM); or saline solution. Intraperitoneal injection of the anti-inflammatory molecule ibuprofen (75mg/kg) does not modify methyl-beta-cyclodextrin-induced tactile hyperalgia.

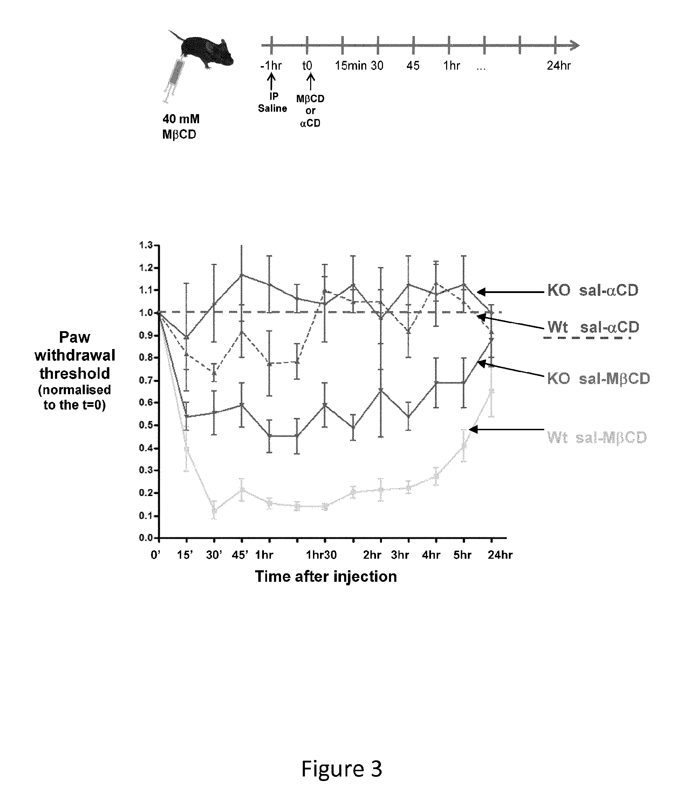

**Figure 3** shows the time course of tactile hyperalgesia measured with von Frey filaments in mice intraplantarly injected with 20 µl of methyl-beta-cyclodextrin, (40mM); or alpha-cyclodextrin (40mM) solution. Alpha-cyclodextrin does not induce tactile hyperalgia.

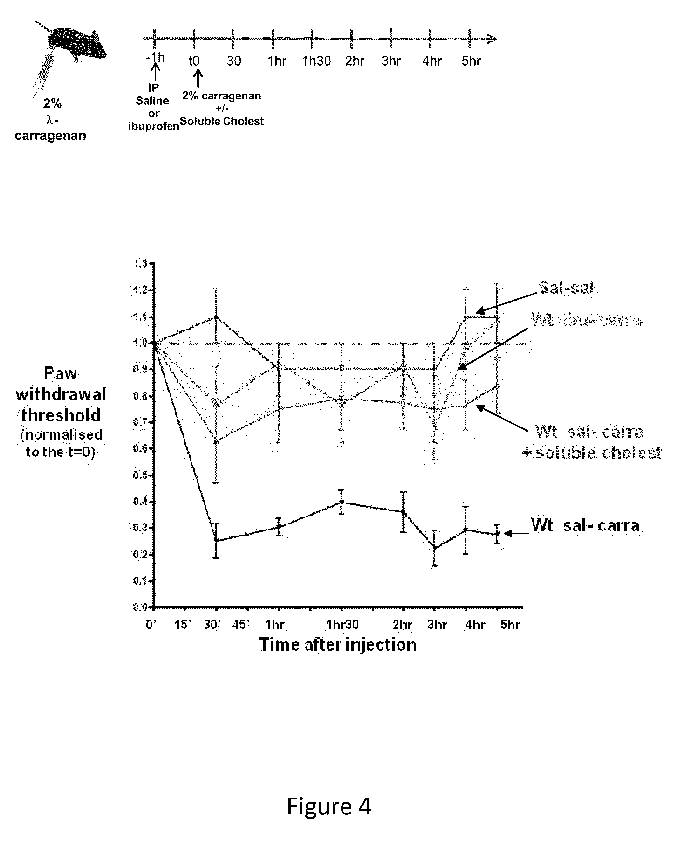

**Figure 4** shows the time course of tactile hyperalgesia measured with von Frey filaments in mice intraplantarly injected with 20 µl of 2% lambda-carrageenan, intraperitoneal injection of ibuprofen reverse lambda-carrageenan-induced tactile hyperalgesia. Intraplantar injection of cholesterol complexed with methyl-beta-cyclodextrin (soluble cholesterol) also reverses lambda-carrageenan-induced tactile hyperalgesia to control levels (saline injected animals).

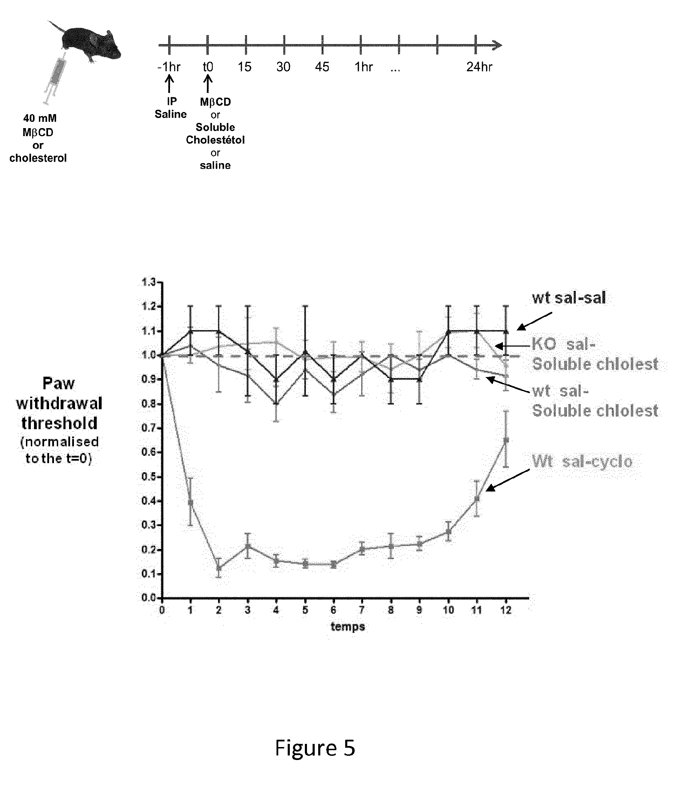

**Figure 5** shows that in non inflamed animals, intraplantar injection of soluble cholesterol does not change mechanical threshold of wild type and Nav1.9 KO mice in physiological non inflamed conditions.

### EXAMPLES

Comparing pain phenotype in wild type (wt) or knocked-out (KO) mice, inventors established the proof of concept that disturbing lipid raft architecture by cholesterol uptake causes Nav1.9 activation which contributes to pain signalling.

Inventors demonstrated that Nav1.9 channels are localised in lipid raft microdomains at the cell membrane of nociceptive neurons of dorsal root ganglia (Figure1). As further explained below, inventors used methyl-beta-cyclodextrin (MBCD) to selectively extract cholesterol from the plasma membrane. They showed that intra plantar injection of 20µl of a 40mM solution of MBCD induces a strong mechanical allodynia as measured with the von Frey filament test (n=7). This MBCD-induced allodynia is markedly reduced in Nav1.9 KO animals (n=6). Inventors showed that this allodynia is not due to an inflammatory process since intra-peritoneal injection of ibuprofen (75mg/kg) did not change mechanical threshold neither in wild type (n=5) nor in Nav1.9 KO (n=10) animals(Figure 2). Moreover, similar injection of 40mM of α-cyclodextrin (α-CD), an inactive analogue of methyl-beta-cyclodextrin that cannot extract cholesterol from membrane, did not induce mechanical allodynia (n=4 both for wild type and Nav1.9 KO animals; Figure 3).
Taken all together these results show that MBCD-induced cholesterol uptake activates Nav1.9 channels and results in mechanical allodynia.
Conversely, inventors showed that topic application of cholesterol inhibits inflammatory evoked pain (n=4; Figure 4), without changing physiological mechanical threshold (n=9 both in wild type and Nav1.9 KO animals; Figure 5).

### Materials & methods

All animals were used in accordance with the European Community guiding in the care and use of animals (86/609/CEE). They were housed under controlled environmental conditions and kept under a 12/12 h light/dark cycle, with food and water *ad libitum.*

### Raft gradient fraction:

Lipid-rich plasma membrane domains were isolated, as detergent-resistant membranes (DRMs), principally by flotation from a dense solution through a discontinuous or continuous density gradient containing the non-ionic detergent Triton X-100.
Freshly dissociated DRG from one mouse were homogenized in 140 µl of lysis buffer with 0.5 % triton X-100 and 1% proteinase inhibitor cocktail (Roche). The homogenate centrifuged at 1,000 g for 10 min at 4°C and the supernatants were incubated 1h on ice, and then subjected to density gradient ultracentrifugation along OptiPrep™ step density gradients.
TLS-55 ultracentrifuge tubes were filled from the bottom to the top as follow: 140 µl of the supernatant was mixed with 280 µl of 60% Optiprep™. To this, 1.6 ml of 30% Optiprep™ (1:1 mix of 60% optiprep solution with lysis buffer) was overlaid, followed by 200 µl of 0% Optiprep™ (lysis buffer only). The tubes were centrifuged at 55 00 rpm (200,000 g) for 4 h at 4°C. Twelve fractions of 150 µl each were gently removed from the top of the tube and individually aliquoted. Fractions were numbered from low (n°1) to high (n°12) density fractions. The fractions were stored at -80°C for up to 6 months.
Fractions were further analysed by western blot. As protein concentration does not provide a true baseline parameter for comparison across fractions, equal sample volume loading was used for the Western blot. This loading method accounts for differential separation of proteins into specific fractions following density ultracentrifugation. From each fraction, an equal sample volume (30 µl) was separated into 4-12% SDS polyacrylamide gels. Protein was transferred onto nitrocellulose membranes. Membranes were incubated with 1:1,000 dilution of an antibody to flotillin 1 (Sigma) to identify the low-density raft fractions. Membranes were additionally probed with a 1:500 dilution of an antibody to TfR (Invitrogen) and with a 1:10000 β-actin (Sigma) to identify non raft fractions. Separately, membranes were probed with a 1:2000 dilution of the Nav1.9 antibody produced in our laboratory. Roche POD Western Chemiluminescent kits were used for protein detection.

### Cyclodextrin and cholesterol manipulations:

Methyl βcyclodextrin or α-cyclodextrin (both from sigma) were diluted in saline buffer prior to injection. Methyl βcyclodextrin /cholesterol complex (40mg of cholesterol/g of complex) also called soluble cholesterol was purchased (from Sigma). All cyclodextrin or cyclodextrin/cholesterol complexes were injected at a 40mM concentration of cyclodextrin.

### Carrageenan model:

Persistent paw inflammation was induced by intraplantar injection of 20 µl of 2% λ-carrageenan (Sigma) in mice. Control animal received only vehicle saline solution (0.9% NaCl).
1hr prior to carrageenan injection, animals received intra peritoneal injection of either saline or ibuprofen (75mg/kg in saline solution).

### Complete Freund's Adjuvent (CFA) induced chronic inflammation:

Chronic inflammation (monoarthritis model) was induced under 2% isoflurane anesthesia by two subcutaneous injections of 15µl around the tibio-tarsal joint of mice with complete Freund adjuvant (CFA), containing 5µg/µl heat-killed *Mycobacterium butyricum* (Becton, Dickinson and cie).

### Behavioral assays

### Von Frey test

Mechanical pain threshold was assessed using von Frey filaments (BioSeb) calibrated from 0.008 to 4.000 g. The filaments were applied five times each, in order of increasing forces, and pressed perpendicularly to the plantar surface of the hindpaw until they bent. The first filament which evoked at least 3/5 response was assigned as the pain threshold in grams.

### Dynamic weight bearing

The animals' weight distribution on the four limbs was assessed using the dynamic weight bearing test (BioSeb). This new incapacitance test consists in a continuous measurement of all pressure points applied by a freely moving animal, allowing a quantitative evaluation of the weight imbalance caused by paw mechanical allodynia. Mice were placed in an 11x11x22 cm cage with a 44x44 sensor cells grid on the floor. The pressure applied on sensor cells by the animal's paws is recorded at a 10 Hz frequency over a 5 min period. Pressure and surface detection thresholds were determined automatically for each animal by the Dynamic weight bearing 1.3.2h software (Bioseb). After the manual attribution of each pressure point to the corresponding paw, the mean weight applied on each paw is calculated. Unilateral pain is finally evaluated trough the ipsi/contralateral hindpaws weight ratio, the weight applied on forepaws, and the percentage of time spent raising ipsilateral hindpaw.

## Claims

1. A lipid inhibiting the Naᵥ1.9 channel activity for use as active ingredient for preventing, alleviating or treating pain in a subject.

2. The lipid according to claim 1, wherein said lipid inhibiting the Naᵥ1.9 channel activity is preferably selected from cholesterol, in particular soluble cholesterol, a sphingolipid and any combination thereof.

3. The lipid according to anyone of claims 1 or 2, wherein pain is selected from an acute pain, a subacute pain and a chronic pain.

4. The lipid according to anyone of claims 1-3, wherein pain is selected from allodynia and hyperalgesia.

5. The lipid according to anyone of claims 1-4, wherein pain is selected from an inflammatory pain; a mechanical heat or cold hypersensitivity; a visceral pain, in particular an enteric pain; and a somatic pain, in particular a trigeminal neuralgia.

6. The lipid according to anyone of claims 3-5, wherein said pain is associated with a gastrointestinal syndrome.

7. The lipid according to anyone of claims 1-6, wherein pain is a pain sensitive to steroidal anti-inflammatory drug (SAID), non steroidal anti-inflammatory drug (NSAID) or opioid, and wherein said pain is to be treated with a combination of said lipid together with a reduced amount of SAID, NSAID or opioid when compared to the administration of SAID, NSAID or opioid alone.

8. The lipid according to anyone of claim 1-7, wherein the subject is resistant to a drug selected from SAID, NSAID and opioid, or suffers from gastrointestinal and/or renal adverse effects induced by said drug.

9. The lipid according to anyone of claim 1-8, wherein the lipid is for cutaneous, subcutaneous, dermic, transdermic, ocular or rectal administration to the subject, preferably for topic administration on an inflammation site.

10. The lipid according to anyone of claim 1-9, wherein the subject is a vertebrate, typically a mammal, preferably a human being.

11. A composition comprising a lipid according to anyone of claims 1 to 10 as an active ingredient and, preferably, a pharmaceutically acceptable carrier.

12. The composition of claim 11, wherein said composition further comprises at least one additional active compound.

13. The composition of claim 12, wherein said at least one additional active compound is an inhibitor of the degradation of a lipid inhibiting the Naᵥ1.9 channel activity, preferably selected from a cholesterol oxidase inhibitor and a sphingomyelinase enzyme.

14. The composition of anyone of claims 11 to 13, wherein said composition is a local analgesic and/or anti-hyperalgesic composition.

15. A kit comprising i) a lipid according anyone of claims 1 to 10 or a composition comprising such a lipid according to anyone of claims 11-14, and ii) at least one additional distinct active compound efficient against pain.
